# EUROPEAN PATENT APPLICATION

(11) **EP 4 443 088 A1**
(43) Date of publication of application: **09.10.2024**
(21) Application number: 22900139.1
(22) Date of filing: 21.10.2022
(51) Int. Cl.: F25D 25/02, F25D 23/12, F25D 11/02, F25D 17/08, A23B 4/06, A23L 13/10, A23L 13/70

(54) **AGING DRAWER FOR REFRIGERATION APPARATUS, AND REFRIGERATION APPARATUS**

(30) Priority: 30.11.2021 CN 202111450276
(71) Applicant: Qingdao Haier Refrigerator Co., Ltd., Laoshan District Qingdao Shandong 266101 (CN); Haier Smart Home Co., Ltd., Qingdao, Shandong 266101 (CN)
(72) Inventor: WANG, Chunli, Qingdao, Shandong 266101 (CN); ZHANG, Hao, Qingdao, Shandong 266101 (CN); CHEN, Tong, Qingdao, Shandong 266101 (CN); CUI, Zhanpeng, Qingdao, Shandong 266101 (CN)
(74) Representative: Winter, Brandl - Partnerschaft mbB
(86) International application number: PCT/CN2022/126736
(87) International publication number: WO 2023/098334

(57) **Abstract**

An aging drawer (1) for a refrigeration apparatus, and a refrigeration apparatus. The aging drawer (1) comprises: a housing (10) and at least one air duct plate (20), which is provided in the housing (10), wherein the housing (10) and the at least one air duct plate (20) define an aging chamber (40) located on an inner side of the air duct plate (20) and at least one air supply duct surrounding at least part of an outer circumferential side of the aging chamber (40), and the aging chamber (40) and the air supply duct (50) communicate with each other by means of a plurality of air supply ports (21) arranged in the air duct plate (20) in a flowing direction of an airflow in the air supply duct (50); and a circulation fan (30), which is configured to controllably propel the airflow in the air supply duct (50) to flow to the aging chamber (40) by means of the plurality of air supply ports (21). An air supply section (51), which is in direct communication with at least the plurality of air supply ports (21), of the air supply duct (50) is configured to taper in the flowing direction of the airflow in the air supply section (51), so that the airflow flows through each position in the section (51) at a uniform flow rate, thereby ensuring that the air supply at each position in the aging chamber (40) in an extension direction of the air supply section (51) is uniform, so as to improve an aging effect.

## Description

### TECHNICAL FIELD

The present application relates to the field of refrigeration apparatus, and in particular, to an aging drawer and refrigerator apparatus with the same.

### BACKGROUND

Aging involves placing acid-drained meat in a chamber with specific temperature, humidity, and airflow to slowly and naturally ferment, ultimately enhancing the depth of flavor, tenderness, flavor, and juiciness of the meat. In the past, the aging drawer is usually standalone, thereby requiring specially temperature control devices and humidity control devices, which makes the structure relatively complex. Moreover, when the volume of food is large, it can lead to uneven airflow over the surface of the food, which affects the aging effect.

### SUMMARY

One objective of the first aspect of the present application is to overcome at least one deficiency of the prior art by providing an aging drawer for refrigeration apparatus with even airflow supply.

A further objective of the first aspect of the present application is to facilitate the structural matching of the aging drawer and the refrigerator apparatus.

Another objective of the present application is to provide a refrigerator apparatus with the aging drawer as described above.

According to the first aspect of the present application, the present application provides an aging drawer for a refrigeration apparatus for maturating objects to be aged therein, the aging drawer comprising: a shell and at least one air duct board provided within the shell, the shell and the at least one air duct board define a maturing chamber on the inside of the air duct board and at least one air supply duct configured to enclose at least part of the periphery outside the maturing chamber, the maturing chamber is connected to the air supply duct through a plurality of air outlets arranged along the direction of airflow within the air supply duct; and a circulation fan, configured to control the airflow within the air supply duct to flow towards the maturing chamber through the plurality of air outlets; wherein at least an air supply section of the air supply duct, which is directly connected to the multiple air outlets, is configured to gradually taper along the direction of airflow therein, thereby ensuring the airflow passes through the air supply section at a uniform speed at different positions.

Further, wherein the air supply section comprises a starting port and an ending port in the direction of the airflow, and the ratio of the cross-sectional areas of the starting port to the ending port is positively correlated with the length of the air supply section.

Further, wherein the air supply duct also comprises a guide air section directly connected to the circulation fan, the guide air section is located upstream of the air supply section.

Further, wherein the circulation fan is located on the rear side of the shell; the air duct board is L-shaped, and the air duct board includes a rear side panel located at the rear within the shell and a lateral side panel located on one lateral side within the shell, the guide air section is defined between the rear side panel and the rear wall of the shell, and the air supply section is defined between the lateral side panel and one lateral wall of the shell; and the air outlets are provided on the lateral side panel.

Further, wherein one lateral wall of the shell extends straight in a front-to-rear direction; and the lateral side panel of the air duct board extends arranged to incline from rear to front and toward the lateral side wall of the shell, thereby causing the air supply section to gradually taper from rear to front.

Further, wherein the angle between the lateral side panel of the air duct board and the lateral wall of the shell ranges between 3° and 15°.

Further, wherein there are two air duct boards, each air duct board and the shell define an air supply duct; and the two air duct boards are symmetrically arranged on the lateral sides within the shell such that the two air supply ducts enclose the lateral sides and the rear side of the maturing chamber.

Further, wherein the rear wall of the shell is provided with a protruding part projecting forwards to form a storage space on the rear side of the protruding part, the storage space is exterior of the shell, and the circulation fan is located within the storage space ; the front side of the protrusion is provided with a return air inlet which allows the airflow flow from the maturing chamber back to the circulation fan, an air outlet is provided on each lateral side of the protrusion, and each of the two air outlets is connected to one of the two air supply ducts respectively.

Further, wherein the circulation fan is a centrifugal fan, centrifugal fan comprise a volute and a centrifugal impeller located within the volute; each lateral side of the volute is provided with an airflow outlet, and each airflow outlet is connected to one of the two air outlets respectively; the top of the volute features an arc-shaped structure protruding upwards in the middle.

According to the second aspect of the present application, the present application provides a refrigeration apparatus comprising: a cabinet that defines a storage space for storing objects; and an aging drawer described above, the aging drawer is configured to mature objects therein, and the aging drawer is provided in the storage compartment.

One objective of the first aspect of this invention is to overcome at least one deficiency of the current technology by providing an aging drawer for refrigeration apparatus that ensures even airflow.

A further objective of the first aspect of this invention is to facilitate the structural compatibility of the aging drawer with the refrigeration apparatus.

The second aspect of this invention aims to provide refrigeration apparatus incorporating the aforementioned aging drawer. According to the first aspect of the invention, it provides an aging drawer for refrigeration apparatus, designed for the aging of items placed within.

In the present application, the aging drawer is applied to refrigeration apparatus, can effectively use the low-temperature environment within the refrigeration apparatus to mature the objects in the aging drawer. There is no need to set up a specialized temperature control device and a humidity control device, simplifying the structure of the aging drawer and reducing the cost of the aging drawer. Moreover, the shell and the at least one air duct board define a maturing chamber on the inside of the air duct board and at least one air supply duct configured to enclose at least part of the periphery outside the maturing chamber. Driven by the circulating fan, the airflow within the air supply duct to flow towards the maturing chamber through the plurality of air outlets. At least an air supply section of the air supply duct, which is directly connected to the multiple air outlets, is configured to gradually taper along the direction of airflow therein, thereby ensuring the airflow passes through the air supply section at a uniform speed at different positions. That is, the airflow speeds in the upstream area, downstream area and central area within the air supply section are balanced. The airflow speed entering the maturing chamber through the air outlets connected to the upstream area of the air supply section, through those connected to the downstream area of the air supply section, and through those connected to the central area of the air supply section are all the same, which ensures uniform air supply throughout the extension direction of the air supply section into the maturing chamber, avoiding the problem of poor aging results caused by inadequate air blowing in some areas of the maturing chamber caused by the gradually reduced airflow speed along its flow direction.

Further, the lateral side wall of the shell is set to extend straight along the direction of front and rear, and only the lateral side panel of the air duct board is arranged to incline from rear to front and toward the lateral side wall. On one hand, the air supply section can be tapered from rear to front, so as to ensure balanced airflow speed within different areas of the air supply section. On the other hand, the outer contour of the shell is more regular, so that it is ease to match the structure of the refrigerator apparatus when the aging drawer is assembled into the refrigeration apparatus, reducing the limitation and waste of space within the refrigeration apparatus.

Specific embodiments of the present application are described in detail below with reference to the accompanying drawings, and thus a person skilled in the art will better understand the foregoing and other objectives, advantages, and features of the present application.

### BRIEF DESCRIPTION OF THE DRAWINGS

Some specific embodiments of the present application are described in detail below in an exemplary rather than restrictive manner with reference to the accompanying drawings. The same reference numerals in the accompanying drawings denote the same or similar components or portions. A person skilled in the art should understand that these accompanying drawings are not necessarily drawn to scale. In the drawings:
FIG. 1 is a schematic structural diagram of an aging drawer according to an embodiment of the present application;
FIG. 2 is a schematic exploded view of the aging drawer according to an embodiment of the present application;
FIG. 3 is a schematic cross-sectional view of the aging drawer according to an embodiment of the present application;
FIG. 4 is a schematic cross-sectional view of the aging drawer from a top view according to an embodiment of the present application;
FIG. 5 is a schematic structural diagram of a shell according to an embodiment of the present application;
FIG. 6 is a schematic structural diagram of a refrigeration apparatus according to an embodiment of the present application.

### DETAILED DESCRIPTION OF EMBODIMENTS

This present application initially provides an aging drawer for refrigeration apparatus, the aging drawer is configured to mature objects to be aged therein. That is to say, the aging drawer of the present application, when applied to refrigeration apparatus, can effectively use the low-temperature environment within the refrigeration apparatus to mature the objects in the aging drawer. There is no need to set up a specialized temperature control device and a humidity control device, simplifying the structure of the aging drawer and reducing the cost of the aging drawer.

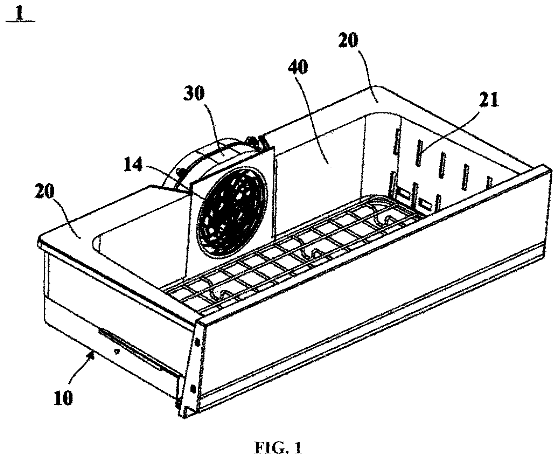

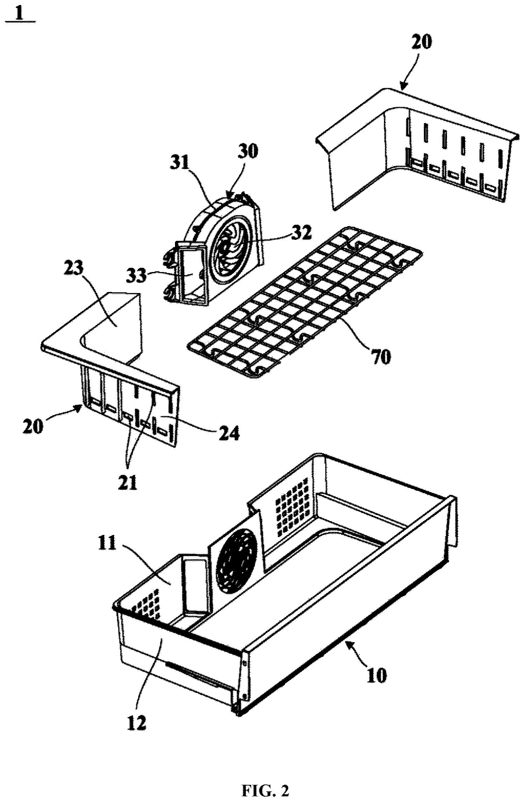

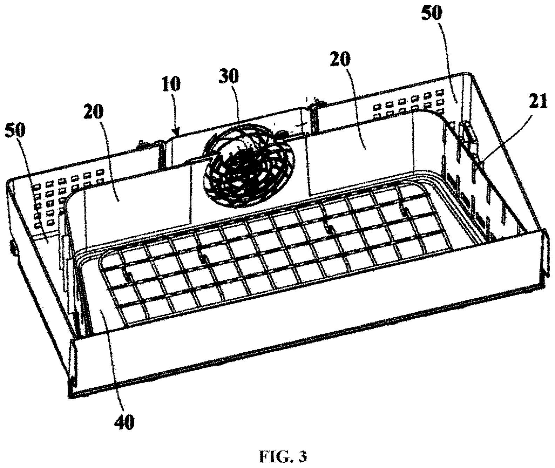

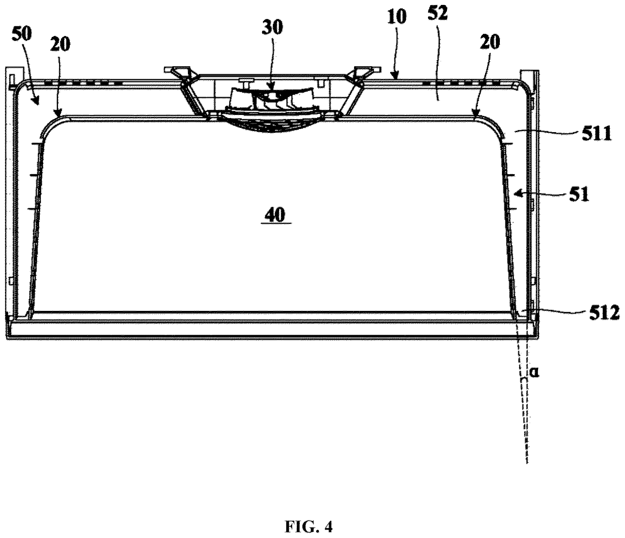

FIG. 1 is a schematic structural diagram of an aging drawer according to an embodiment of the present application. FIG. 2 is a schematic exploded view of the aging drawer according to an embodiment of the present application. FIG. 3 is a schematic cross-sectional view of the aging drawer according to an embodiment of the present application. FIG. 4 is a schematic cross-sectional view of the aging drawer from a top view according to an embodiment of the present application. Referring to FIGS 1 to 4, the aging drawer 1 comprises a shell 10, at least one air duct board 20 provided within the shell 10, and a circulation fan 30.

A maturing chamber 40 and at least one air supply duct 50 are defined between the shell 10 and the at least one air duct board 20, the maturing chamber 40 is positioned on the inside of the air duct board 20, and the air supply duct 50 is configured to enclose at least a portion of the circumferential outside of the maturing chamber 40. The duct plate 20 is provided with a plurality of air outlets 21, the plurality of air outlets 21 being arranged along the direction of air flow within the air supply duct 50, and the maturing chamber 40 is connected to the air supply duct 50 through the plurality of air outlets 21. The air supply duct 50 is disposed around the circumferential periphery of the maturing chamber 40 so as to facilitate air supply from the circumferential direction of the maturing chamber 40 to the maturing chamber 40, which provides a wider range of air supply and facilitates uniform air supply of the objects to be aged in the maturing chamber 40.

The circulation fan 30 is configured to control the airflow within the air supply duct 50 to flow towards the maturing chamber 40 through multiple air outlets 21 and the airflow within the maturing chamber 40 to return back to the air supply duct 50 through the return air inlet 14. Thereby, the airflow circulates between the air supply duct 50 and the maturing chamber 40, driven by the circulation fan 30. In the present application, there is an additional circulation fan 30 set within the aging drawer 1, so that the air can be continuously blow into the maturing chamber 40 without affected by the state of the fan within the refrigeration apparatus to achieve better aging results. Specifically, the circulation fan 30 can draw air from inside the maturing chamber 40 and send it towards the air supply duct 50, causing the airflow within the air supply duct 50 to move towards the maturing chamber 40 under negative pressure; or the circulation fan 30 can also draw air from the air supply duct 50 and send it towards the maturing chamber 40, causing the airflow inside the maturing chamber 40 to move towards the air supply duct 50 under negative pressure.

Specifically, an air supply section 51 of the air supply duct 50, which is directly connected to multiple air outlets 21, is configured to taper along the direction of airflow therein, thereby ensuring the airflow passes through the air supply section 51 at a uniform speed at different positions. It should be noted that the air supply section 51 of the air supply duct 50 directly connected to multiple air outlets 21 refers to the section corresponding to the position on the air duct board 20 where the air outlets 21 are located. However the other sections of the air supply duct 50 corresponding to the position of the air duct board 20 without air outlet 21 are not considered as the air supply section 51.

In the present application, the air supply section 51 of the air supply duct 50 formed within the aging drawer 1 is set to taper along the direction of airflow within the air supply section 51, so that the airflow passes through different positions of the air supply section 51 at a uniform speed. That is, the airflow speeds in the upstream area, downstream area and central area within the air supply section 51 are balanced. The airflow speed entering the maturing chamber 40 through the air outlets 21 connected to the upstream area of the air supply section 51, through those connected to the downstream area of the air supply section 51, and through those connected to the central area of the air supply section 51 are all the same, which ensures uniform air supply throughout the extension direction of the air supply section 51 into the maturing chamber 40, avoiding the problem of poor aging results caused by inadequate air blowing in some areas of the maturing chamber 40 caused by the gradually reduced airflow speed along its flow direction.

In some embodiments, the air supply section 51 comprises a starting port 511 located at the upstream and an ending port 512 located at the downstream in the direction of the airflow. The ratio of the cross-sectional areas of the starting port 511 to the ending port 512 is positively correlated with the length of the air supply section 51. That is, the longer the length of the air supply section 51, the greater the ratio of the cross-sectional areas between the starting port 511 and the ending port 512, indicating a greater degree of tapering and more pronounced effect, ensuring the airflow near the ending port 512 has sufficient speed. The shorter the length of the air supply section 51, the smaller the ratio of the cross-sectional areas between the starting port 511 and the ending port 512, indicating a lesser degree of tapering and less pronounced effect, preventing the issue of increased airflow resistance and uneven flow speed caused by too rapid a decrease in the cross-sectional area of section 51.

In the present application, matching the degree of tapering of the air supply section 51 along the direction of air flow within the air supply section 51 with the length of the air supply section 51, making it easier to achieve a balanced airflow speed at each of the air outlets 21.

In some embodiments, the air supply duct 50 also includes a guide air section 52 directly connected to the circulation fan 30, the guide air section 52 is located upstream of the air supply section 51. That is, the circulation fan 30 drives the airflow to circulate by blowing air into the air supply duct 50.

In some embodiments, the circulation fan 30 is positioned on the rear side of the shell 10, which makes it less likely for users to perceive the noise generated by the operation of the circulation fan 30, and facilitates the use of one circulation fan 30 to supply air into multiple air supply ducts 50 to improve the uniformity of air supply into the maturing chamber 40.

Further, the air duct board 20 is L-shaped and includes a rear side panel 23 located at the rear within the shell 10 and a lateral side panel 24 located on one lateral side within the shell 10. A guide air section 52 is defined between the rear side panel 23 and a rear wall 11 of the shell 10, and the air supply section 51 is defined between the lateral side panel 24 and one lateral wall 12 of the shell 10. Thus, the guide air section 52 extends laterally, and the air supply section 51 extends in the front-to-rear direction, with the airflow direction within the air supply section 51 moving from rear to front, and the cross-sectional area of the air supply section 51 tapering from rear to front.

Moreover, the air outlets 21 are provided on the lateral side panel 24. Specifically, multiple air outlets 21 are distributed on the lateral side panel 24 along the front-to-rear direction so as to facilitate air supply into the maturing chamber 40 from various positions in the front and back directions, which improves the uniformity of air supply into the maturing chamber 40 in the front-to-rear direction.

It's understood that there are multiple ways to achieve the tapering of the air supply section 51 along the direction of airflow within. Preferably, in some embodiments, one of the lateral side walls 12 of the shell 10 extends straight in the front-to-rear direction, the lateral side panel 24 of the air duct board 20 extends from rear to front while gradually near the lateral side wall 12, thus the air supply section 51 tapers from rear to front.

In the present application, the lateral side wall 12 of the shell 10 is set to extend straight along the direction of front and rear, and only the lateral side panel 24 of the air duct board 20 is arranged to incline from rear to front and toward the lateral side wall 12. On one hand, this meets the requirement for the air supply section 51 to taper from rear to front, so as to ensure balanced airflow speed within different areas of the air supply section 51. On the other hand, the outer contour of the shell 10 is more regular, so that it is ease to match the structure of the refrigerator apparatus when the aging drawer 1 is assembled into the refrigeration apparatus, reducing the limitation and waste of space within the refrigeration apparatus.

In some embodiments, the angle α between the lateral side panel 24 of the air duct board 20 and one lateral side wall 12 of the shell 10 ranges between 3° to 15°. For instance, the angle α between the lateral side panel 24 and the lateral side wall 12 can be 3°, 5°, 7°, 9°, 11°, 13°, or 15°. If the angle α between the lateral side panel 24 and the lateral side wall 12 is too large, the degree of tapering for the air supply section 51 is more obvious, and the lateral side panel 24 and the lateral side wall 12 generate higher resistance to airflow and greater loss of airflow speed. Conversely, if the angle α is too small, the tapering of the air supply section 51 is too gentle, which is not conducive to achieving the goal of balancing airflow speed through the shape of the tapering of the air supply section 51.

In some embodiments, there are two air duct boards 20, an air supply duct 50 is defined between one air duct plate 20 and the shell 10, and another air supply duct 50 is defined between another air duct plate 20 and the shell 10. The two air duct boards 20 are symmetrically arranged on the lateral sides within the shell 10 such that the two air supply ducts 50 enclose the lateral sides and the rear side of the maturing chamber 40. As a result, air can be blown into the maturing chamber 40 from both lateral sides simultaneously through the symmetrically arranged two air supply ducts 50, thereby improving the uniformity of airflow across the lateral direction of the maturing chamber 40 and thus enhancing the aging effect of the objects to be matured.

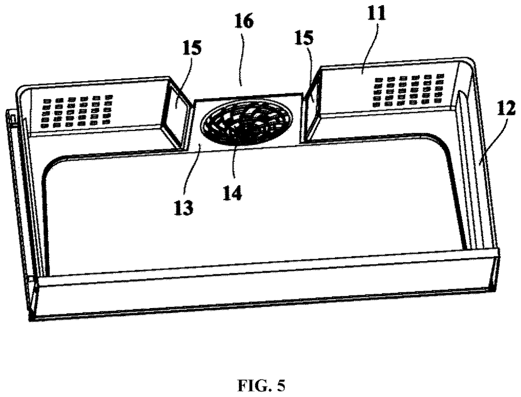

Figure 5 is a schematic structural diagram of the shell according to an embodiment of the present application. In some embodiments, the rear wall 11 of the shell 10 is provided with a protruding part 13 projecting forwards to form a storage space 16 on the rear side of the protruding part 13, the storage space 16 is exterior of the shell 10, and the circulation fan 30 is located within the storage space 16. That is, the circulation fan 30 is mounted on the exterior of the shell 10, the circulation fan 30 is separated from the maturing chamber 40 via the wall of the shell 10, there is no need to set additional partitions or components within the shell 10, thereby simplifying the structure and assembly process of the aging drawer 1, and improving assembly efficiency.

In some embodiments, the front side of the protrusion 13 is provided with a return air inlet 14 which allows the airflow flow from the maturing chamber 40 back to the circulation fan 30. An air outlet 15 is provided on each lateral side of the protrusion 13, and each of the two air outlets 15 is connected to one of the two air supply ducts 50 respectively. Consequently, the airflow within the maturing chamber 40 can flow through the return air inlet 14 to the circulation fan 30 and then return into the two air supply ducts 50 respectively under the drive of the circulation fan 30.

Further, the circulation fan 30 is a centrifugal fan. The circulation fan 30 comprises a volute 31 and a centrifugal impeller 32 positioned within the volute 31. Each lateral side of the volute 31 is provided with an airflow outlet 33, and each of the two airflow outlets 33 is connected to one of the two air outlets 15 to supply airflow towards the two air supply ducts 40 respectively.

In the present application, a centrifugal fan supplies airflow to both air supply ducts 40 simultaneously, thereby facilitating airflow to flow into the maturing chamber 40 from both lateral sides of the maturing chamber 40 at the same time, which not only achieves the effect of uniform air supply, but also simplifies the structure of the aging drawer 1.

Furthermore, the top of the volute 31 features an arc-shaped structure protruding upwards in the middle. That is, the top of the volute 31 forms an arc-shaped wind guiding structure within to guide the airflow towards the two airflow outlets 33 on the lateral sides, thereby reducing airflow resistance and loss of air volume.

Moreover, the bottom of the volute 31 is nearly flat or forms an arc-shaped structure recessed downward in the middle, with the depth of the recess at the bottom of the volute 31 being less than the protrusion at the top, to minimize the space occupied by the circulation fan 30 as much as possible.

In some embodiments, a rack 70 is provided in the maturing chamber 40 for placing objects to be matured. The rack 70 is set spaced apart from the bottom wall of the shell 10 to leave a space for airflow underneath the rack 70 to facilitate air flow to the underside of objects placed on the rack 70, thus ensuring that the underside of the objects can achieve a drying effect as soon as possible.

Specifically, the rack 70 is provided with a plurality of hollowed mesh holes, the hollowed mesh holes reduce the contact area between the objects to be matured and the rack 70, thereby preventing parts of the surface of the objects from spoiling due to lack of airflow contact.

Further, the height of the bottom edge of the return air inlet 14 is lower than the height of the rack 70, which benefits the airflow moving beneath the rack 70 to return to the air supply duct 50 through the bottom area of the return air inlet 14, thereby reducing the resistance of the bottom airflow and further enhancing the speed of airflow movement.

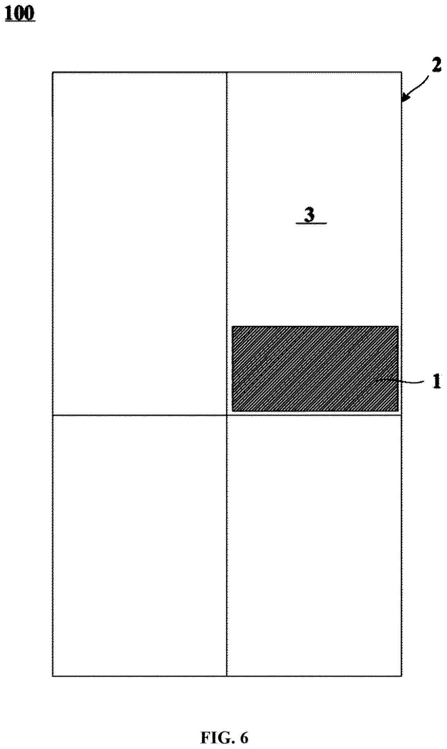

The present application also provides a refrigeration apparatus 100, and FIG. 6 is a schematic structural diagram of a refrigeration apparatus according to an embodiment of the present application. The refrigeration apparatus 100 includes a cabinet 2, and the cabinet 2 is defined a storage compartment 3 for storing objects. In particular, the refrigeration apparatus 100 further includes the aging drawer 1 described in any of the above embodiments. The aging drawer 1 is configured to mature objects therein, and the aging drawer 1 is provided in the storage compartment 3. The refrigeration apparatus 100 integrated with the aging drawer 1 not only retains the traditional function of preserving objects in a low-temperature environment but also possesses the function of maturating, thereby enriching the functionalities of the refrigeration apparatus 100.

Specifically, the aging drawer 1 with an opening at the top thereof can be slid in and out of the storage compartment 3, thereby making the aging drawer 1 convenient for users to place and retrieve objects.

Furthermore, the storage compartment 3 can serve as a refrigeration chamber with a storage temperature range between 0°C to 8°C, which is suitable for the aging temperature requirements of the objects to be matured. Moreover, the humidity level within the refrigeration chamber usually exceeds that of the indoor environment, which is more suitable for the humidity requirements of the aging process. Even if the humidity within the aging drawer 1 is not appropriate, it can be easily adjusted through the airflow within the refrigeration chamber. Therefore, the aging drawer 1 is provided within the refrigeration chamber, which can fully utilize the inherent temperature and humidity conditions of the refrigeration chamber, and significantly reduce costs.

Additionally, the cabinet 2 may also define a freezer compartment and/or a variable-temperature compartment. The refrigeration chamber, freezer compartment, and variable-temperature compartment can each be selectively opened or closed by doors pivotally mounted on the front side of the cabinet 2.

In some embodiments, the refrigeration apparatus 100 can be a standard refrigeration and freezing device. For example, the refrigeration apparatus 1 can also be a refrigerator, freezer, or cool box used for domestic or commercial purposes.

In other embodiments, the refrigeration apparatus 100 can also configured as other types of refrigeration devices such as sideboard cabinet, wine cabinet, and freezer, which require higher precision in temperature and humidity control.

Additionally, it should be noted that terms such as "horizontal," "vertical," "front," "rear," "top," and "bottom" used to describe orientations or positional relationships in the embodiments of the present application are based on the actual usage state of the aging drawer 1. These terms are used solely for the convenience of describing and understanding the technical embodiments of the present application, and are not intended to indicate or imply that the devices or components must possess specific orientations or be constructed and operated in specific orientations; hence, they should not be construed as limiting the scope of the present application.

Moreover, it is necessary to clarify that, unless otherwise clearly specified and defined, the terms "mounted", "connected", "connection", "fixed", and the like should be understood in a broad sense. For example, unless otherwise expressly defined, a connection may be a fixed connection, a detachable connection, or an integrated connection; a direct connection, or an indirect connection through an intermediate medium; or interaction between two elements. A person of ordinary skill in the art may understand specific meanings of the above terms in the present application based on specific situations.

Thus, a person skilled in the art should be aware that although a plurality of exemplary embodiments of the present application have been shown and described in detail in this description, many other variations or modifications conforming to the principle of the present application can still be determined or deduced directly according to the content disclosed in the present application, without departing from the spirit and scope of the present application. Therefore, the scope of the present application shall be understood and considered as covering all such other variations or modifications.

## Claims

1. An aging drawer for refrigeration apparatus, configured to mature objects to be aged therein, wherein the aging drawer comprising:
a shell and at least one air duct board provided within the shell, the shell and the at least one air duct board define a maturing chamber on the inside of the air duct board and at least one air supply duct configured to enclose at least part of the periphery outside the maturing chamber, the maturing chamber is connected to the air supply duct through a plurality of air outlets arranged along the direction of airflow within the air supply duct; and
a circulation fan, configured to control the airflow within the air supply duct to flow towards the maturing chamber through the plurality of air outlets; wherein
at least an air supply section of the air supply duct, which is directly connected to the multiple air outlets, is configured to gradually taper along the direction of airflow therein, thereby ensuring the airflow passes through the air supply section at a uniform speed at all positions.

2. The aging drawer according to claim 1, wherein the air supply section comprises an starting port and an ending port in the direction of the airflow, and the ratio of the cross-sectional areas of the starting port to the ending port is positively correlated with the length of the air supply section.

3. The aging drawer according to claim 2, wherein the air supply duct also comprises a guide air section directly connected to the circulation fan, the guide air section is located upstream of the air supply section.

4. The aging drawer according to claim 3, wherein
the circulation fan is located on the rear side of the shell;
the air duct board is L-shaped, and the air duct board includes a rear side panel located at the rear within the shell and a lateral side panel located on one lateral side within the shell, the guide air section is defined between the rear side panel and the rear wall of the shell, and the air supply section is defined between the lateral side panel and one lateral wall of the shell; and
the air outlets are provided on the lateral side panel.

5. The aging drawer according to claim 4, wherein:
one lateral wall of the shell extends straight in a front-to-rear direction; and
the lateral side panel of the air duct board extends arranged to incline from rear to front and toward the lateral side wall of the shell, thereby causing the air supply section to gradually taper from rear to front.

6. The aging drawer according to claim 5, wherein:
the angle between the lateral side panel of the air duct board and the lateral wall of the shell ranges between 3° and 15°.

7. The aging drawer according to any one of claims 4 to 6, wherein:
there are two air duct boards, each air duct board and the shell define an air supply duct; and
the two air duct boards are symmetrically arranged on the lateral sides within the shell such that the two air supply ducts enclose the lateral sides and the rear side of the maturing chamber.

8. The aging drawer according to claim 7, wherein:
the rear wall of the shell is provided with a protruding part projecting forwards to form a storage space on the rear side of the protruding part, the storage space is exterior of the shell, and the circulation fan is located within the storage space ;
the front side of the protrusion is provided with a return air inlet which allows the airflow flow from the maturing chamber back to the circulation fan, an air outlet is provided on each lateral side of the protrusion, and each of the two air outlets is connected to one of the two air supply ducts respectively.

9. The aging drawer according to claim 8, wherein:
the circulation fan is a centrifugal fan, centrifugal fan comprise a volute and a centrifugal impeller located within the volute;
each lateral side of the volute is provided with an airflow outlet, and each airflow outlet is connected to one of the two air outlets respectively;
the top of the volute features an arc-shaped structure protruding upwards in the middle.

10. A refrigeration apparatus comprising:
a cabinet that defines a storage space for storing objects; and
an aging drawer according to any one of claims 1-9, the aging drawer is configured to mature objects therein, and the aging drawer is provided in the storage compartment.
